Europäisches Patentamt

European Patent Office

Office européen des brevets

(11) Publication number: **0 420 053 A1**

# EUROPEAN PATENT APPLICATION

(21) Application number: **90118151.1**

(22) Date of filing: **21.09.90**

(51) Int. Cl.5: **G01N 33/545**, G01N 33/549, G01N 33/548, G01N 33/546

(30) Priority: **26.09.89 US 412963**

(43) Date of publication of application:
**03.04.91 Bulletin 91/14**

(84) Designated Contracting States:
**DE FR GB IT NL**

(71) Applicant: **W.R. Grace & Co.-Conn.**
**Grace Plaza, 1114 Ave. of the Americas**
**New York New York 10036(US)**

(72) Inventor: **Rudolph, Julie Liao**
**186 Bellows Hill Road**
**Carlisle, MA 01741(US)**

(74) Representative: **UEXKÜLL & STOLBERG**
**Patentanwälte**
**Beselerstrasse 4**
**W-2000 Hamburg 52(DE)**

(54) **Improved solid assay support systems.**

(57) An improved solid assay support system is prepared by entrapping or immobilizing bioaffinity agent-coated particles on a porous, absorbent flat sheet material which has a protein non-adsorptive polyurethane polymeric coating thereon. The support system is useful in many diagnostic or other analytical assays, including those of the half-sandwich, full sandwich, antibody capture, competitive or non-competitive type or types.

EP 0 420 053 A1

## IMPROVED SOLID ASSAY SUPPORT SYSTEMS

This is a continuation-in-part of copending U.S. Serial No. 07/235,411 (Rudolph et al.), filed August 22, 1988.

### BACKGROUND OF THE INVENTION

This invention relates to the use of solid supports for conducting diagnostic or other analytical assays. More specifically, an improved solid support system has been created which utilizes solid particles coated with antibody or another bioaffinity agent, which coated solid particles are immobilized on a porous, absorbent material. The support system of this invention display high signal intensity, thereby increasing assay sensitivity, and resist rapid signal fading. The support system can be used in various assay formats and for a variety of diagnostic and other purposes. The support system can be used with half-sandwich, full-sandwich, antibody capture, competitive and non-competitive immunoassays, nucleic acid hybridizations, and the like.

Immunoassays have conventionally been conducted using a microporous matrix as a support for a bioactive agent, which detects the presence of a particular desired protein (the "target" protein). These bioactive agents, usually antibodies to the target protein, are immobilized in some manner on the matrix for use in the diagnostic testing. The microporous matrix frequently is a microporous membrane, such as nylon, upon which the bioaffinity agent is immobilized by passive adsorption. Next, a blocking step is required for the prevention of nonspecific binding of either the target protein or a recognition protein or agent to the target protein. Nonspecific binding across the entire membrane renders the assay inaccurate and unreadible. The blocking step conventionally comprises thoroughly coating the membrane with a protein in order to fill, or block, the nonspecific binding sites available on the support surface. Alternatively, the microporous matrix previously has been blocked by coating with a polymer resistant to nonspecific protein binding, as described above and as taught in U.S. 4,794,090 (Parham et al.)

Solid particles, such as polystyrene latex beads, have long been known in the diagnostics field, particularly as modified by coating with proteins such as antibodies. Most commonly, latex beads are used in agglutination assays. In such tests, the latex particles are coated with antibodies (or other proteins), and a drop of coated particles is mixed with a drop of sample. If the sample contains the antigen, the latex particles will clump together to become agglutinated.

Other assay formats also have used solid particles. U.S. 4,347,312 (Brown et al.) discloses an assay in which antibodies are covalently bound or adsorbed to surfaces such as the surface of porous beads. U.S. 4,663,277 (Wang) discloses a viral assay using a mobile solid phase comprising a dispersion of microspheres coated with antiviral antibody and an extended solid phase (i.e., a dipstick) coated with antiviral antibody; detection of bound microspheres on the extended solid phase is the basis of the assay.

Cellulosic and other porous absorbent materials also have been used in the diagnostics field. Chen et al., "An Internal Clock Reaction Used in a One-Step Enzyme Immuno-chromatographic Assay of Theophylline in Whole Blood," Clin. Chem. Vol. 33, pp. 1521-25 (1987), discloses an enzyme assay using a chromatographic paper test strip having an antibody and glucose oxidase immobilized at the bottom. The strip is placed in a liquid sample, which is drawn up through the strip by wicking action. Color is generated as a clearly visible mobile front on the paper, with the height of the resulting colored area being proportional to the concentration of antigen in the sample. U.S. 4,168,146 (Grubb et al.) teaches the use of a capillarity-possessing porous carrier to which antibodies are bound. The porous carrier may be a cellulose fiber-containing material.

Conducting assays using these prior methods typically requires multiple process steps and reagent mixtures. In addition, the colorimetric results achieved using a horseradish peroxidase system with prior art assay supports are subject to rapid fading, foreclosing the possibility of confirming the test results without repeating the assay. The solid support system of this invention provides easy to use assay supports which yield a high intensity color signal resistant to rapid fading, whether the system is based on alkaline phosphatase, horseradish peroxidase or colloidal gold.

### SUMMARY OF THE INVENTION

The assay support system of this invention comprises a porous, absorbent support material on which solid particles coated with a bioaffinity agent are immobilized. This solid support system is useful in various assay formats and may be used with labelling systems such as enzyme or colloidal gold colorimetric systems, or radionuclide reagents. It is expected to be useful with fluorescent labelling systems as well.

It is a primary object of this invention to provide a solid assay support system which produces a stable visible or measurable assay result. For colorimetric assays, it is intended that an intense color signal be obtained which will resist fading for at least 24 hours.

Another object is to provide an assay support system in which the bioaffinity agent can be rapidly applied and affixed to the support. A related object is the reduction or avoidance of leaching of the bioaffinity agent from the assay support system during assay procedures.

A further object is to provide an assay support system on which an intense signal can be generated, yielding an assay with high sensitivity. The invention is thus intended to provide a highly sensitive assay which can detect low levels of analyte, such as antibody or antigen. It is also intended that the signal appear in a fixed signal generation area of the assay support system.

It is an additional object to provide an assay support system having a very low nonspecific binding affinity for bioreagents or other proteins which would interfere with the assay results.


## DETAILED DESCRIPTION OF THE INVENTION


A novel assay support system is provided in which a bioaffinity agent is immobilized onto particles or beads which, in turn, are entrapped or immobilized on a porous, absorbent support material which has been pretreated with a polyurethane prepolymer to render it resistant to nonspecific protein binding. The support material preferably is cellulosic and may be fiber-containing or may be a microporous membrane. This support system can be used in a wide variety of assays, depending on the bioaffinity agent which is selected. The support system is advantageously used with colorimetric labelling systems employing enzyme or colloidal gold reagents, but also may be used with fluorescent or radionuclide labelling systems. The support system is adaptable for use in formats such as strips, dipsticks, chips, microtiter plates, etc.

Support Material - The underlying support material used in this invention is a porous, absorbent flat sheet material. Cellulosic materials are particularly suitable. A variety of papers or other fiber-containing cellulose materials may be used, although non-fibrous materials may be used as well. The material may be microporous or macroporous, woven or non-woven, having an average pore diameter of between about 0.1 and about 30.0 microns, preferably about 10.0 to about 20.0 microns.

The support material must be an absorbent material for test liquid transport in order to ensure adequate contact with the bioaffinity agent immobilized on the support. That is, the material should be capable of wicking the test liquid from the container or holder in which it is placed. In formats such as strips or dipsticks, it is preferred that the material be sufficiently rigid that it does not collapse upon wetting. However, some flexibility is permitted and this invention does not require particularly rigid supports in most embodiments.

The term "cellulose" as used herein is intended to encompass cellulose and modified cellulose materials, such as cellulose esters (e.g., cellulose acetate), cellulose ethers (e.g., methylcellulose), and rayon. Chromatographic papers and filter papers, for example Whatman filter paper, may be used. Circuit board material is a preferred choice, as is cellulosic battery separator material. For example, cellulose circuit board material OA84 (Grace Specialty Chemicals Co., W. R. Grace & Co.-Conn.) and DAWEB™ cellulose battery separator material (Grace Specialty Chemicals Co., W. R. Grace & Co.-Conn.) are both preferred for use as the support material in this invention. Both materials are made from wood pulp.

Non-cellulosic materials of suitable absorbency and stiffness may be used in this invention. For example, porous materials of nylon, polyvinyl alcohol, polyester, polyvinylchloride, polyethylene, polypropylene, polyvinylidene fluoride, etc. are suitable.

Polymeric Pretreatment - The support material preferably is provided with a protein non-adsorptive polyurethane polymer precoating in order to reduce or eliminate nonspecific binding of protein onto the assay surface. This precoating eliminates the need for separate blocking steps after immobilization of the bioaffinity agent-coated particles. In addition, the urethane coating allows antibodies and other bioaffinity agents to be stably immobilized on the support material. The result is good retention of antibody or other bioaffinity agent, with the product having a suitably long shelf life. It has been found that the support materials prepared according to this invention exhibit excellent retention of the bioaffinity agent-coated particles, with very little leaching of the particles or bioaffinity agent from the assay support system during assay procedures. No aging of the precoated support material is necessary prior to immobilization of the bioaffinity agent-coated particles.

The polyurethane polymer used for this precoating preferably is one formed by polymerization of a prepolymer prepared by capping a polyoxyalkylene polyol with a polyisocyanate compound, such that the prepolymer has a reaction functionality greater than two. Suitable polyurethane prepolymers of this type are disclosed in U.S. 4,137,200 (Wood et al.). Particularly preferred is HYPOL™ 6100 polyurethane prepolymer (Grace Specialty Chemicals Co., W. R. Grace & Co.-Conn.). Other suitable polyurethane prepolymers are disclosed in USSN 130,826, "Biocompatible Polyurea-Urethane Hydrated Polymers" (Braatz et al.), filed December 9, 1987. These latter prepolymers are available under the trademark BIOPOL from Grace Specialty Chemicals Co., W. R. Grace & Co.-Conn.

A solution is prepared comprising the polyurethane prepolymer in a volatile organic solvent, (for example, acetone, alcohols, chlorinated hydrocarbons, etc.) in a concentration of about 0.1 to about 20.0 percent prepolymer. A surfactant also may be present. The porous support material is contacted with the solution and then dried. Detailed procedures for coating membranes in this fashion are given in U.S. 4,794,090 (Parham et al.), which is incorporated herein by reference.

Alternatively, the porous material may be coated with a solution of an aqueous-based polyurethane polymer in a volatile organic solvent, thus forming a polyurethane polymer coating as described in the parent case, which is incorporated herein by reference. The material is contacted with a solution comprising an aqueous-based polyurethane polymer and a volatile organic solvent (for example, acetone, alcohols, chlorinated hydrocarbons, etc.) in a concentration of about 0.1 to about 20.0 percent polymer. A non-ionic surfactant also may be present. The porous support material is contacted with the solution and then dried. The polymer may be any of the elastomers described in U.S. 4,442,259 (Isgur et al.). Most preferably, the coating is prepared by treatment with DARATHANE™ WB-22 polyurethane elastomer (Grace Specialty Chemicals Co., W. R. Grace & Co.-Conn.), which is an aliphatic urethane polymer in an aqueous solution of about 40% solids in ethanol. A dual polymer coating comprising DARATHANE elastomer and a second polyurethane polymer may be used. For example, a solution of DARATHANE WB-22 elastomer (4%) and HYPOL 6100 prepolymer (1%) in acetone may be used to coat the porous material.

Bioaffinity Agents - This invention finds wide application in the area of immunoassays and other assays involving bioaffinity agents and/or biological test samples. For example, the agent frequently will be an antibody to the target protein of the assay, most preferably a monoclonel antibody. Where the assay support system is used for antibody screening, the bioaffinity agent will be the antigen, such as a protein or glycoprotein, to which an antibody is sought. The assay support system also can be used as a nucleic acid probe, with the appropriate ribonucleic acid or deoxyribonucleic acid as the bioaffinity agent. The choice of bioaffinity agent will depend on the assay in which the support system is to be used, and is not limited to those agents specifically disclosed or discussed here.

Particulate Material - The desired bioaffinity agent or agents are coated onto particulate materials for use in the assay support system of this invention. The particulate materials may be porous or nonporous. Microspheres or beads in the range of about 0.1 to about 10 microns in diameter are preferred, with about 1.0 to about 3.0 microns most preferred. The particles conveniently may be composed of polystyrene latex or modified latex, for example, styrene/butadiene, carboxylate-modified latex, styrene/divinylbenzene, vinyltoluene/tertiary-butyl styrene, styrene/vinyltoluene, amino-modified latex, amide-modified latex, hydroxyl-modified latex, polyvinyltoluene, polymethyl-methacrylate and vinyl benzyl chloride. Dyed particles may be used. The particles, particularly styrene/butadiene particles, may be radiolabelled.

Alternatively, other particulate materials may be used. Particles of any material which is considered suitable for use in antibody-antigen immunoassays or other assays employing bioaffinity agents will be suitable here as well. For example, silicas or polyurethane-coated silicas will be suitable. Magnetic particles also may be used. These may be tiny crystals of a magnetic or paramagnetic material (e.g., the ferrimagnetic mineral magnelite) which are dispersed in polystyrene or modified polystyrene spheres.

Proteins (e.g., antibodies) or other bioaffinity agents as discussed above are coated onto the particles. This may be by absorption or adsorption, particularly in the case of latex beads or silica. Covalent coupling may be desired, particularly in the case of modified latex beads, such as with carboxylated or aminated latex surfaces.

Standard methods of coating the particles may be used, for example, as directed by the supplier of the particles. Latex beads may be coated by passive adsorption by stirring a latex suspension with a buffer solution containing the bioactive agent, and then separating the latex from unadsorbed bioactive agent. The coated particles may be resuspended in buffer. Alternatively, the antibody or other agent can be covalently coupled to the particles, either directly or through the use of coupling agents or spacers. Non-latex particles may be coated in similar or other convenient manners and suspended in buffer. Any buffer or other solution may be used in which the bioactive agent is stable.

Assay Support System - The bioactive agent-coated particles are then immobilized on the treated porous absorbent support material. A small amount of the bioactive agent-coated particle suspension is applied to the treated support material and dried. Preferably, the suspension is applied as a sample dot, line, symbol or other shape to form the test portion of the assay support system. In preparing the improved assay support systems of this invention, only minor amounts of the bioactive agent-coated particles are needed, for example, about 1.0 to about 10.0 μl, preferably about 3.0 μl.

Drying may be at room temperature or higher, although care should be taken not to expose the support system to temperatures which will be likely to denature the biologically active agent. As examples of suitable temperatures and drying times, the material may be dried at 80°C for about ten minutes or at 50°C for about ten to thirty minutes or at room temperature for at least about four hours. For purposes of this description, the porous, absorbent support material which has been treated with polymer and onto which a bioactive agent has been immobilized will be referred to as an "assay support system".

Permanent, covalent attachment of the biologically active agent-coated particles to the support material probably is not believed to be achieved by this process. Rather, it is believed that they become well entrapped in the structure of the absorbent support material. Immobilization of the particles onto the porous support material is sufficient for the uses contemplated by this invention. Dissolution and removal of the particles or bioactive agent from the support material requires washing for longer periods and/or under harsher conditions than typically encountered during the assay procedures. For example, the support system may be serially contacted with test or sample fluid and with reagent solutions, with simple rinsing steps in-between. It has been demonstrated that sufficient quantities of the bioactive agent remain immobilized on the assay support material during these steps to yield a sensitive and reliable assay support system.

In an alternative embodiment, permanent covalent attachment of the bioactive agent-coated particles to the support material may be desired. The antibody or other agent coated on the particles may be bound to the polymer-treated support material by the action of a cross linking agent. Amine crosslinking agents such as glutaraldehyde react with the amine groups of the bioactive agent and the amine groups of the polymer surface and will be suitable for this purpose. Other suitable amine cross linking agents include di- or trifunctional amine reactive agents such as diepoxides, di- or triisocyanates, disuccinimidyl suberate or dimethyl suberimidate and the like. Alternatively, the amino groups on the polymer surface can be attached to the carboxylate groups of the bioactive agent using crosslinking agents such as carbodiimides or carbonyldiimidazoles, for example. The crosslinking agent may be added before, with or after application of the coated particles. This embodiment will be particularly useful in those applications where it is desired that the bioactive agent be more tightly bound to the assay support material. However, the assay signal may not be as strong in this embodiment.

Assay support systems prepared according to this invention may be used to test various fluids for the presence of particular target proteins. For example, bodily fluids such as urine, plasma, serum, whole blood or milk may be tested. The bioactive agent is sufficiently immobilized on the support material to resist displacement by other proteins which may be present during the assay. This is true even where test fluids such as blood or blood components are used, which contain significant quantities of background protein in addition to the target protein. These assay support systems also may be used to test for the presence of proteins in other types of fluids, for analytical purposes other than diagnosis.

The assay support systems of this invention may be used directly following immobilization of the bioactive agent. The need for a protein blocking step has been eliminated. The assay support systems of this invention are not susceptible to nonspecific protein binding, so long as the support remains wet during the assay, which discourages proteins from being absorbed into the support material in sufficient quantities to be detected or to interfere with the assay. That is, only the target protein will be bound to the support, via the immobilized bioaffinity agent, during the assay. The binding of the target protein will occur only in the area of the immobilized bioactive agent by virtue of the unique binding sites associated therewith. Thus, interference from other proteins present in the test fluid has been eliminated, as has possible ambiguity of test results caused by indiscriminate binding of the target protein.

Assay Formats - The assay support systems of this invention can be used in a variety of formats. For example, they may be used in assays for processing samples and reagents via flow through, vacuum-driven flow, diffusion or upward elution set-ups. The size and shape of the support system will be selected on the basis of convenience in the selected format. In addition, the configuration of immobilized bioactive agent-coated particles on the support material may be varied as necessary or desirable for the assay format, appearing, for example, as a dot, a line, a symbol, etc. Various assay types--half-sandwich, full sandwich, competitive, etc.--may be conducted using any of these formats.

Similarly, numerous fluids, solutions or suspensions can be used as the test fluid. For example, this assay support system may be used to test most bodily fluids, e.g., urine, blood, blood serum, plasma, sputum, etc. Other fluids such as conditioned medium, cell conjugate, etc. may be used.

The assay support system of this invention may be used according to conventional assay methods and procedures. According to one procedure, using a colorimetric assay, the test fluid is brought into contact with the assay support system, optionally followed by rinsing. Next, a solution of an appropriate recognition conjugate contacts the support system, again optionally followed by rinsing. Finally, a substrate solution contacts the system which will cause a color change in the area of the bound bioaffinity agent/target protein/recognition conjugate complex. The appearance of this color change indicates the assay is positive for the target protein; lack of color change indicates the absence of the target protein in the test fluid. Using the assay support of this invention, a color change may be apparent within about 10.0 to about 20.0 seconds. The time required for appearance of color will depend on such factors as target protein concentration, recognition conjugate, temperature and the like, and can be adjusted accordingly.

According to a second procedure, the test fluid and solution of the appropriate recognition conjugate are mixed together and the mixture is brought into contact with the assay support system. The system is incubated for five to ten minutes. Insulation may be followed by rinsing and blotting. The color change occurs as described above. Other procedures and variations for using the assay support system of this invention may be used as desired.

The above description is written in terms of a full-sandwich enzymatic colorimetric assay, in which the recognition conjugate comprises an enzyme, such as horseradish peroxidase or alkaline phosphatase, which causes a visually apparent color change under positive test conditions. Colorimetric assays based on colloidal gold labelling systems are also appropriate. However, the utility of the assay support system of this invention is not limited to colorimetric assays. These assay systems may be used with any convenient indicator system. For example, the recognition antibody may be radiolabelled for use in a radioassay or may be labelled with a fluorescing compound for use in a fluorescence assay. In any event, the assay support system is contacted with the test sample or fluid and appropriate reagents (e.g., recognition conjugate and substrate), and then observed for the presence or absence of a signal generated in the area in which the bioaffinity agent-coated beads are immobilized.

As stated above, various support system shapes or configurations may be used. More specifically, in one embodiment, the assay support system may be cut into conveniently sized strips with a dot of coated particles at or near one end, to be used in a strip or dipstick format assay. Alternatively, the support system may be cut into small discs or chips each having a dot of coated particles at or near the center. The coated particles most conveniently are dotted onto the support material, but other configurations or methods of application to the support may be used as desired or convenient. Still another suitable embodiment is a multi-well device or microtiter plate in which dots of coated particles are placed in wells on the treated support material. In each of these embodiments, the support system is contacted with a sample of test fluid and the appropriate assay reagents for conduct of the assay.

The test sample and reagent liquids must be in sufficient contact with the assay support system to allow absorption of the liquid(s) in the area of immobilized bioaffinity agent-coated particles, i.e., the test area. It is absorption at this site which is critical during the assay. This may be achieved by immersing the test area in the liquids. Alternatively, the assay support system may be brought into contact with a small quantity of test and/or reagent liquid in such a manner that sufficient quantities of liquid wick or elute past the test area to thoroughly wet the test area with the appropriate liquid. The test area (i.e., signal generation area) remains fixed and immobile on the assay support system, notwithstanding that in certain embodiments the liquid sample and reagents may be eluted through that area by wicking action.

The signal generated during the assay will vary in intensity and durability according to the concentration of target protein in the sample. The intensity of the signal can be purposely varied by adjusting the concentration of tracer, or recognition conjugate. The signal can be maintained for certain periods at room temperature (e.g., 3 days) or for longer periods under refrigeration (e.g., one week).

More than one dot of coated particles may be used on each assay support. In this manner, positive controls can be added to the assay support material by selecting appropriate bioactive agent-coated particles. For example, antibody to the recognition agent used in the assay (e.g., antibody to the enzyme used in a colorimetric assay) may be coated onto latex particles and deposited on the support material for use as a control to detect the presence of active recognition agent (e.g., enzyme). Other controls or additional test dots may be included.

The examples which follow are given for illustrative purposes and are not meant to limit the invention described herein. The following abbreviations have been used throughout in describing the invention.

AP - alkaline phosphatase

°C - degrees Centigrade
HCG - human chorionic gonadotropin
HRP - horseradish peroxidase
IgG - immunoglobulin G
mg - milligram(s)
ml - milliliter(s)
mm - millimeter(s)
ng - nanogram(s)
$\mu$g - microgram(s)
$\mu$l - microliter
PBS - phosphate buffered saline
% - percent
TMB - tetramethyl benzidine
v - volume
w - weight

## EXAMPLE I

DawebTM D-70 cellulose battery separator material (Grace Specialty Chemicals Co., W. R. Grace & Co.-Conn.) was placed in a solution of HYPOL 6100TM polyurethane prepolymer (Grace Specialty Chemicals Co., W. R. Grace & Co.-Conn.) in acetone (2% w/v) containing 0.2% TRITONTM X-100 surfactant (Sigma Chemical Co.) under constant agitation for 30 minutes. The material was removed from the solution and air dried briefly, followed by drying at 50°C overnight or at 80°C for one hour; both drying conditions were found to be suitable. This was designated Support Material A.

## EXAMPLE II

DAWEB D-70 cellulose battery separator material was treated as in Example I except that BIOPOLTM polyurethane prepolymer (Grace Specialty Chemicals Co., W. R. Grace & Co.-Conn.) was used. This was designated Support Material B.

## EXAMPLE III

DAWEB D-70 cellulose battery separator material was treated as in Example I except that a mixture (1/1.4%, w/w) of HYPOL 6100 polyurethane prepolymer and DARATHANETM WB-22 polyurethane polymer (Grace Specialty Chemicals Co., W. R. Grace & Co.-Conn.) was used. This was designated Support Material C.

## EXAMPLE IV

Cellulose circuit board material OA84 (Grace Specialty Chemicals Co., W. R. Grace & Co.-Conn.) was treated according to the procedures of Example I. This was designated Support Material D.

## EXAMPLE V

Cellulose circuit board material OA84 was treated according to the procedures of Example II. This was designated Support Material E.

EXAMPLE VI

Cellulose circuit board material OA84 was treated according to the procedures of Example III. This was designated Support Material F.

EXAMPLE VII

Cellulose circuit board material OA84 was placed in a solution of HYPOL 6100 polyurethane prepolymer (1% w/v) and DARATHANE WB-22 polyurethane polymer (2% w/v) in acetone under constant agitation for one hour. The treated material was removed from the solution and dried at 50°C for 2 hours. The dried material next was treated in the same prepolymer/polymer solution with the addition of 0.2% TRITON X-100 surfactant (v/v). The twice-treated material was removed and dried at 50°C overnight, at 80°C overnight, or at 80°C for one hour; all three drying conditions were found to be suitable. This was designated Support Material G.

EXAMPLE VIII

Whatman paper was treated according to the procedures of Example VII. This was designated Support Material H.

EXAMPLE IX

Support Materials A-F from Examples I-VIII were cut into 5mm x 50 mm strips. Glycoprotein CD4 was passively adsorbed onto 1.0, 2.0 or 3.0 μm polystyrene latex beads by dissolving it in 0.1 M borate buffer (pH 8.0), adding to the latex bead suspension and stirring, followed by removal of unadsorbed glycoprotein CD4. The coated beads were resuspended in borate buffer or PBS. Next, 3-5 μl of the coated latex bead suspension were deposited near one end of each strip and dried.

To run a half-sandwich assay, each strip was placed in a small test tube containing 100 μl anti-CD4 antibody-horseradish peroxidase ("HRP") conjugate solution with the latex dot close to that end of strip immersed in the test liquid. Two minutes were allowed for absorption of the liquid by the strips. Each strip was removed from its test tube and rinsed thoroughly with PBS. The rinsed strips were well blotted on adsorbent towel.

Each strip was placed in a clean test tube containing 100 isl substrate solution (tetramethyl benzidine ("TMB") and hydrogen peroxide) and incubated for at least 5 minutes. In each case, a blue dot on a white strip was observed. The strips retained their color signal for at least 24 hours.

EXAMPLE X

The procedures of Example IX were repeated for Support Materials A-H except that goat anti-rabbit antibody IgG was coated onto the latex beads. Each strip was incubated first in rabbit IgG for five minutes, and then in anti-rabbit IgG-HRP conjugate for five minutes. The strips were rinsed, blotted and treated with substrate as in Example IX. Similar results were obtained, with the blue color signal remaining visible for at least 24 hours.

EXAMPLE XI

Support materials A-G were cut into 5 mm x 100 mm strips. A particle suspension was prepared by adding mg of polystyrene latex (1 micron) to 1.0 ml human IgG solution (1.0 mg/ml in borate buffer) and stirring, followed by separation of coated beads from unbound IgG. The coated latex beads were re-suspended in PBS. About 5 $\mu$l of the IgG-coated latex particle suspension were applied to each strip at about 10 mm from one end of the strip, and dried for 30 minutes at 50° C or 10 minutes at 80° C.

Colloidal gold-goat anti-human IgG conjugate (10 $\mu$l) was mixed with PBS (90 $\mu$l) in small test tubes. The strips were placed in the test tubes with the coated latex particle dot at the bottom, either immersed in or above the liquid, and incubated for 5-10 minutes. A pink dot appeared on each white strip.

### EXAMPLE XII

Support materials A-F and H were cut into strips and treated with human IgG-coated latex beads as in Example XI. Goat anti-human IgG-alkaline phosphatase ("AP") conjugate was diluted (1/1000) with PBS, and 100 $\mu$l aliquots placed in small test tubes. Strips were placed in the test tubes with the coated latex particle dot contacting the liquid and incubated for 5 minutes. The strips were removed, washed with PBS and blotted on absorbent towels. A drop (30 $\mu$l) of indoxyl phosphate was placed on the dot end of the strip for 5 minutes. (As an alternative, the strip can be placed in a test tube containing the indoxyl phosphate substrate.) The strips were washed thoroughly. On each strip, a blue dot appeared on a white background.

### EXAMPLE XIII

Support Materials A-F and H were cut into 5 mm x 100 mm strips. An antibody-particle suspension was prepared as described in Example IX using polystyrene latex beads (1 micron and 2 micron beads) and mouse anti-HCG IgG. About 3 $\mu$l of the suspension were applied to each strip at about 10 mm from one end of the strip and dried for 30 minutes at 50° C.

HCG-containing samples (100 $\mu$IU in 100 $\mu$l either PBS or urine) were mixed with anti-HCG IgG-gold conjugate in small test tubes. The strips were placed in the test tubes with the dotted end in the liquid and incubated for 5-10 minutes. No rinsing was done in this example. Pink dots appeared on the white strips to indicate that the samples were positive for HCG. The color was retained indefinitely without fading.

### EXAMPLE XIV

Support Materials A-C were cut into strips and treated as in Example XIII. HCG-containing samples (100 $\mu$IU HCG in 100 $\mu$l PBS) were mixed with anti-HCG IgG-alkaline phosphatase conjugate in small test tubes. Strips were placed in the test tubes with the coated latex particle dot contacting the liquid and incubated for 5 minutes. The strips were removed, washed with PBS and blotted on absorbent towels. A drop (30 $\mu$l) of indoxyl phosphate was placed on the dot end of the strip for 5 minutes. (As an alternative, the strip can be placed in a test tube containing the indoxyl phosphate substrate.) On each strip, a blue dot appeared on a white background.

### EXAMPLE XV

Support Materials D, E, F and H were cut into one-inch diameter discs. An antibody-particle suspension was prepared by mixing mouse anti-HCG IgG with polystyrene latex beads (1 micron). This suspension was applied in 5 $\mu$l aliquots to the center of each disc and the discs were dried at 50° C for 20 minutes.

Each disc was placed on an absorbent pad and good contact between the two was maintained by means of a plastic holder. HCG samples (100 $\mu$IU HCG in 100 $\mu$l PBS) were mixed with anti-HCG IgG-gold conjugate and applied to the top surface of the assay support systems, allowing the liquid to drain through. After two minutes incubation time, the assay support systems were washed with 1-2 ml tap water. The positive samples were indicated by a pink dot on a white background on each of the discs.

EP 0 420 053 A1

## EXAMPLE XVI

The procedures of Example XV were repeated, substituting anti-HCG IgG-AP conjugate for the anti-HCG IgG-gold conjugate. The positive samples were indicated by a blue dot on a white background on each of the discs.

## EXAMPLE XVII

Support Material G was cut into 5 mm x 90 mm strips. A suspension of glycoprotein CD4 coated polystyrene latex beads (1-3 microns) was prepared according to the general procedures of Example IX, and 3 $\mu$l of the suspension deposited on each strip about 5-10 mm from one end of the strip. The strips were dried at 50° C for 30 minutes.

The strips were placed in small test tubes containing 100 $\mu$l of sample in PBS buffer (either rabbit anti-CD4 serum or purified anti-CD4 antibody), with the latex dot immersed in the liquid or slightly above the liquid. Two to ten minutes were allowed for absorption of the liquid by the strips. The strips were removed, rinsed and blotted, then placed in small test tubes containing the tracer solution (goat anti-rabbit IgG-HRP conjugate. After 2-5 minutes incubation at room temperature, the strips were removed and washed thoroughly with PBS. The strips were blotted as dry as possible without rubbing the latex beads off, then placed in test tubes containing 100 $\mu$l of substrate solution (TMB and hydrogen peroxide). Blue dots developed on the white strips within 10 minutes for serum samples containing 0.1 $\mu$g/ml anti-CD4 protein. The dots remained blue for a week under refrigeration.

## EXAMPLE XVIII

Strips of Support Material G were treated with glycoprotein CD4-coated latex beads as in Example XVII. The same assay was conducted according to the following alternative procedures. Sample (rabbit anti-CD4 serum) and tracer (goat anti-rabbit IgG-HRP conjugate) were mixed together and 200 $\mu$l placed in small test tubes with the strips. The strips were incubated in the liquid for 10 minutes, then removed, rinsed and blotted. The strips were placed in substrate as in Example XVII. Blue dots developed as in Example XVII.

## EXAMPLE XIX

Support Material G cut into 5 mm x 90 mm strips. A suspension of glycoprotein CD4 coated polystyrene latex beads was prepared according to the general procedures of Example IX, and 3-5 $\mu$l of the suspension deposited near the end of each strip. The strips were dried for 30 minutes of 50° C.

Rabbit serum being screened to determine if it contained anti-CD4 antibody was mixed with tracer (goat anti-rabbit IgG-HRP conjugate). The strips were placed in the sample-tracer mixtures and incubated for 5-10 minutes, then washed and blotted. The strips were placed in test tubes containing 100 $\mu$l of substrate solution (TMB and hydrogen peroxide) for up to 10 minutes. Blue signals appeared within 10 minutes, depending on the presence and concentration of the antibody in each serum sample.

## EXAMPLE XX

The procedures of Example XIX were repeated with the substitution of monkey plasma containing anti-CD4 antibody as the sample and goat anti-human IgG-HRP conjugate. Similar results were obtained.

## EXAMPLE XXI

10

The procedures of Example XIX were repeated coating the latex particles with goat anti-rabbit IgG (instead of glycoprotein CD4). The sample (analyte) contained rabbit IgG at concentrations of 50.0 ng/ml, 10.0 ng/nl, 5.0 ng/ml, 1.0 ng/ml, 0.5 ng/ml, and 0.0 ng/ml. The tracer was goat anti-rabbit IgG-HRP conjugate.

The strip assay of the invention was conducted as follows:

The analyte and the tracer (100.0 µl each) were mixed in a small test tube. A strip with the dried latex dot was placed in each test tube with the dot slightly above the liquid level and incubated for 10 minutes. The strips were removed, rinsed with PBS and blotted well. The strips then were placed in second test tubes containing 100.0 µl TMB-peroxide substrate solution. The blue color appeared within one minute for samples containing rabbit IgG at concentrations above 1.0 ng/ml. The signal color was quantified on a Hunter reflectometer as the measurement (DE) of color difference between the signal dot on the strip and a white tile standard. The color on a dot measuring >40(DE) remained visible for over 48 hours.

The microtiter plate assay, used for comparison purposes, was run according to standard procedures, using the reagents described above; A microtiter plate was coated with goat anti-rabbit IgG overnight and blocked with 0.1% casein. The analyte and tracer solutions were incubated in the individual well of the plate (100.0 µl each per well). The liquid was decanted and the wells washed three times with PBS. Next, 100.0 µl TMB substrate solution was placed in each well and incubated for 20 minutes. The color of each well was read on a Dynatech microtiter plate reader at wavelength 630 nm for blue color intensity.

The results are shown in Table I. The assay using the strips of this invention was found to be up to about 10 times more sensitive than using microtiter plate technology in terms of the detection limit of the assay. The strips of this invention can detect IgG at 0.1 ng/ml, whereas the plates require 1.0 to 5.0 ng/ml before a detectable signal can be seen.

The principles, preferred embodiments and modes of operation of the present invention have been described in the foregoing specification. The invention which is intended to be protected herein, however, is not to be construed as limited to the particular forms disclosed, since these are to be regarded as illustative, rather than restrictive. Variations and changes may be made by those skilled in the art without departing from the spirit of the invention.

TABLE I

| Rabbit IgG (ng/ml) | Test Strip | | | Microtiter Plate | | |
|---|---|---|---|---|---|---|
| | Signal Reading (DE) | Signal-Noise Dif. (DE) [1] | Signal/Noise Ratio[2] | Signal Reading (A, 630 nm) | Signal-Noise Dif. (A) [1] | Signal/Noise Ratio[2] |
| 0.0 | 12.5[3] | -- | 1.0 | .227[3] | -- | 1.0 |
| 0.1 | 22.5 | 10.0 | 1.8 | .196 | -- | 1.0 |
| 0.5 | 26.7 | 14.2 | 2.1 | .215 | -- | 1.0 |
| 1.0 | 31.2 | 18.7 | 2.5 | .226 | -- | 1.0 |
| 5.0 | 46.4 | 33.9 | 3.7 | .332 | .105 | 1.5 |
| 10.0 | 56.7 | 44.2 | 4.5 | .503 | .276 | 2.2 |
| 50.0 | 62.0 | 49.5 | 5.0 | 1.230 | 1.003 | 5.4 |

1 - The strip signal-noise difference is found by subtracting the signal reading at 0.0 ng/ml from each other signal reading to determine the reading actually attributable to the assay signal.

2 - The signal.noise ratio is found by dividing the signal reading by the noise level.

3 - Noise level.

## Claims

1. An assay support system comprising a porous, absorbent flat sheet support material having a protein non-adsorptive polyurethane polymeric coating thereon, with a plurality of particles or beads coated with bioaffinity agents entrapped or immobilized on said support material.

2. The assay support system of Claim 1 in which said support material is cellulose or a modified cellulosic material.

3. The assay support system of Claim 2 in which said cellulose or modified cellulosic material is battery separator material, circuit board material, filter paper or chromatographic paper.

4. The assay support system of Claim 1 in which said support material is porous nylon, polyvinyl alcohol, polyester, polyvinylchloride, polyethylene, polypropylene or polyvinylidene fluoride.

5. The assay support system of Claims 1 to 4 in which said polyurethane polymeric coating is formed by polymerization of a prepolymer prepared by capping a polyoxyalkylene polyol with a polyisocyanate compound, said prepolymer having a reaction functionality greater than two.

6. The assay support system of Claims 1 to 5 in which said bioaffinity agent is antigen, polyclonal antibody, monoclonal antibody, protein, glycoprotein, or DNA or RNA probe.

7. A method of using an assay support system according to claims 1 to 6 which comprises a porous, absorbent support material having a protein non-adsorptive polyurethane polymeric coating thereon, and a plurality of bioaf finity agent-coated particles or beads entrapped or immobilized on said support material, comprising contacting said assay support system with test fluid, appropriate recognition conjugate and substrate, and observing the presence or absence of a signal generated in the area of said entrapped or immobilized bioaffinity agent-coated particles or beads.

8. Use according to Claim 7 in which said test fluid is mixed with said recognition conjugate and substrate prior to contacting said assay support system for a one-step assay.

9. Use According to Claim 7 in which said assay support system first contacts said test fluid and then contacts a mixture of appropriate recognition conjugate and substrate solution for a two-step assay.

10. The method of Claim 7 in which said assay support system first contacts said test fluid, next contacts a solution of an appropriate recognition conjugate, and then contacts a substrate solution for a three-step assay.

11. A process for preparing an assay support system according to claims 1 to 6 comprising:

(a) treating a porous, absorbent flat sheet support material to form a protein non-adsorptive polyurethane polymeric coating thereon,

(b) coating particulate material with bioactive agent,

(c) entrapping or immobilizing the bioactive agent-coated particulate material on the treated support material, and

(d) drying.

# EUROPEAN SEARCH REPORT

Application Number

**EP 90 11 8151**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| P,X | EP-A-0 376 135   (W.R. GRACE & CO.-CONN.)<br>* The entire document *<br>– – – | 1,4,5-11 | G 01 N 33/545<br>G 01 N 33/549<br>G 01 N 33/548 |
| Y | | 2,3 | G 01 N 33/546 |
| P,X | EP-A-0 355 687   (W.R. GRACE & CO.-CONN.)<br>* The entire document *<br>– – – | 1-11 | |
| D,Y | US-A-4 794 090   (M.E. PARHAM et al.)<br>* The entire document *<br>– – – | 1-11 | |
| A | EP-A-0 312 135   (FINDLEY ADHESIVES INC.)<br>* The entire document *<br>– – – – – | 1-11 | |

TECHNICAL FIELDS
SEARCHED (Int. Cl.5)

G 01 N

The present search report has been drawn up for all claims

| Place of search | Date of completion of search | Examiner |
|---|---|---|
| The Hague | 18 December 90 | DOEPFER K-P. |

CATEGORY OF CITED DOCUMENTS

X : particularly relevant if taken alone
Y : particularly relevant if combined with another
document of the same category
A : technological background
O : non-written disclosure
P : intermediate document
T : theory or principle underlying the invention

E : earlier patent document, but published on, or after
the filing date
D : document cited in the application
L : document cited for other reasons

 

& : member of the same patent family, corresponding
document